# EUROPÄISCHE PATENTSCHRIFT

(11) **EP 3 097 403 B1**
(45) Veröffentlichungstag und Bekanntmachung des Hinweises auf die Patenterteilung: **06.05.2020**
(21) Anmeldenummer: 15701007.5
(22) Anmeldetag: 22.01.2015
(51) Int. Cl.: G01N 21/3504, G01N 21/05, G01N 21/03, G01N 33/00

(54) **VORRICHTUNG ZUR BESTIMMUNG DER KONZENTRATION ZUMINDEST EINES GASES IN EINEM PROBENGASSTROM MITTELS INFRAROTABSORPTIONSSPEKTROSKOPIE**
DEVICE FOR DETERMINING THE CONCENTRATION OF AT LEAST ONE GAS IN A SAMPLE GAS FLOW BY MEANS OF INFRARED ABSORPTION SPECTROSCOPY
DISPOSITIF POUR LA DÉTERMINATION DE LA CONCENTRATION D'AU MOINS UN GAZ DANS UN COURANT DE GAZ ÉCHANTILLON AU MOYEN DE LA SPECTROSCOPIE PAR ABSORPTION INFRAROUGE

(30) Priorität: 22.01.2014 DE 102014100691
(43) Veröffentlichungstag der Anmeldung: 30.11.2016
(73) Patentinhaber: AVL Emission Test Systems GmbH, 41460 Neuss (DE)
(72) Erfinder: FETZNER, Stephan, 76694 Forst (DE); BRUNNER, Raimund, 79206 Breisach (DE); ULMER, Ulrich, 79106 Freiburg (DE)
(74) Vertreter: terpatent Patentanwälte ter Smitten Eberlein-Van Hoof Rütten Daubert
(86) Internationale Anmeldenummer: PCT/EP2015/051205
(87) Internationale Veröffentlichungsnummer: WO 2015/110503

(56) Entgegenhaltungen:
- EP-A1- 1 724 567
- EP-A2- 1 596 184
- WO-A1-2010/146079
- GB-A- 2 255 194
- JP-A- 2000 002 656
- JP-A- 2000 180 356

## Beschreibung

Die Erfindung betrifft eine Vorrichtung zur Bestimmung der Konzentration zumindest eines Gases in einem Probengasstrom mittels Infrarotabsorptionsspektroskopie mit einer Infrarotstrahlungsquelle, deren Strahlung durch einen Raum einer Gaszelle führbar ist, einem Probengasstrom, welcher in den Raum und durch die Strahlung leitbar ist, einem Detektor, auf welchen die aus dem Raum austretende Strahlung leitbar ist und mittels dessen das Absorptionsspektrum der austretenden Strahlung ermittelbar ist und einer Heizquelle, über die der Probengasstrom auf eine Solltemperatur aufgeheizt wird.

Die Infrarotspektroskopie ist zur Bestimmung der Konzentration von einzelnen Gaskomponenten bekannt. Die am weitesten verbreiteten Verfahren sind das Fourier Transform Infrarotspektrometer oder das nicht dispersive Infrarotspektrometer. Mit der Entwicklung von kompakten, leistungsstarken Halbleiterlasern etablieren sich zunehmend Gasanalysatoren auf Basis der Laserspektroskopie. Neue Lasertypen wie Quantenkaskadenlaser revolutionieren die Laserspektroskopie im mittleren Infrarotbereich.

Alle diese Analysemethoden beruhen darauf, dass bei Bestrahlung eines Probengases mit Infrarotstrahlen bestimmte Frequenzbereiche absorbiert werden. Die Infrarotstrahlung liegt dabei in dem Bereich des Schwingungsniveaus von Molekülbindungen, die durch die Absorption zum Schwingen angeregt werden. Voraussetzung hierfür ist ein vorhandenes oder im Molekül erzeugbares Dipolmoment. Die verschiedenen Schwingungszustände verursachen Absorptionsverluste der Infrarotstrahlung unterschiedlicher optischer Frequenzen. Das Spektrum in der Transmission enthält somit einzelne für das Gas charakteristische Absorptionslinien, so dass das Probengas auf das Vorhandensein konkreter Moleküle untersucht und deren Konzentration im Probengas bestimmt werden kann..

Mit einem Quantenkaskadenlaser können insbesondere im Abgas von Verbrennungsmotoren vorhandene Schadstoffmoleküle, wie Distickstoffmonoxid, Stickstoffmonoxid, Stickstoffdioxid, Kohlendioxid, Kohlenmonoxid und Ammoniak ermittelt und deren Konzentration bestimmt werden.

Übliche laserspektroskopische Systeme weisen einen Laser als Strahlungsquelle auf, dessen Strahlung über einen optischen Pfad in eine Gaszelle geleitet wird. In dieser Gaszelle wird der Strahl über eine geeignete Spiegelkonfiguration mehrfach reflektiert. Gleichzeitig wird in diese Gaszelle ein Probengasstrom geleitet, durch den die Strahlung des Lasers dringt und dort für eine Anregung der zur optischen Frequenz korrespondierenden Moleküle sorgt. Durch diese Anregung wird Energie der jeweiligen Frequenz absorbiert. Die Intensität des transmittierten Strahls nimmt an dieser Stelle im Spektrum ab. Die Absorption selbst erfolgt nicht exakt scharf, sondern unterliegt einer Verbreiterung aufgrund von Temperatur- und Druckänderungen. Der auf diese Weise in seinem Spektrum veränderte Strahl verlässt die Messzelle und trifft auf einen Detektor, über den das veränderte Frequenzband ausgewertet wird und so auf das Vorhandensein bestimmter Stoffe und deren Konzentrationen geschlossen werden kann. Die Förderung des Probengasstroms erfolgt üblicherweise über eine nachgeschaltete Vakuumpumpe.

Bei der Bestimmung der Konzentration wird die Absorptionscharakteristik im Spektrum ausgewertet bzw. analysiert. Diese Charakteristik wird im Allgemeinen als Linienspektrum der absorbierenden Gase bezeichnet. Es hat sich jedoch gezeigt, dass die Linienform in diesem Spektrum vom Druck und der Temperatur abhängig ist. Diese Parameter müssen daher für eine Auswertung entweder konstant gehalten oder müssen kontinuierlich messtechnisch erfasst und verrechnet werden. Daher wird zur Erhöhung der Messgenauigkeit das Gas konditioniert und Druck und Temperatur konstant gehalten. Für die Temperierung muss sowohl die Messzelle als auch die Zuleitung des Messgases beheizt werden. Ein Auftreten von Temperaturgradienten muss daher innerhalb der gesamten Probenahme verhindert werden, wodurch einerseits Gasverschleppungseffekte vermieden werden und andererseits thermische Turbulenzen vermieden werden, die das Absorptionsverhalten der Laserstrahlung beim Durchgang durch das Medium störend beeinflussen.

Um eine solche Konditionierung des Probengases durchzuführen, ist es bekannt, die Gaszelle und das Probengas vorab auf eine bestimmte Temperatur aufzuheizen. Zum Vorwärmen des Probengases werden beispielsweise Heizschläuche verwendet, wie dies in der EP 2 388 570 A1 beschrieben wird. Die Messzelle wird ebenso wie das Probengas im Zufuhrschlauch auf beispielsweise 191°C erwärmt. Mit dieser Ausführung können die Messergebnisse zwar verbessert werden, jedoch besteht das Problem, dass zwei verschiedene Temperaturfühler und Temperaturregler verwendet werden, wodurch Messfehler durch auftretende Temperaturgradienten zwischen Gaszelle und Probengasstrom nicht vollständig ausgeschlossen werden können.

Des Weiteren offenbart die JP 2000002656 einen Gasanalysator, der eine Probengasleitung aufweist, die auf einer Heizplatte zwischen der Gaszelle und einem spiralförmig verlaufenden Abschnitt der Probengasleitung, welcher vor dem Einlass in die Gaszelle angeordnet ist, angeordnet ist. Die Gaszelle mit dem Probengasstrom sind in einer gemeinsamen thermischen Isolierung angeordnet.

Die EP 1 724 567 A1 offenbart eine Vorrichtung gemäß dem Oberbegriff des Anspruchs 1 mit einer Absorptionszelle, an der ein Gasstrom an einem Heizer vorbeigeführt wird, über den die Zelle temperiert wird. Anschließend gelangt der Gasstrom zur Messung in die Zelle. Die EP 1 596 184 A2 offenbart ein Infrarotspektrometer, bei dem die Gaszelle eine Isolierung sowie einen Heizer und einen Peliterkühler aufweist. Die Probengaskanäle ragen durch den Heizer.

Daher stellt sich die Aufgabe, eine Vorrichtung zur Bestimmung der Konzentration zumindest eines Gases in einem Probengasstrom mittels Infrarotabsorption zu schaffen, mit der die Messergebnisse im Vergleich zu bekannten Ausführungen weiter verbessert werden, indem Temperaturgradienten zwischen dem Probengas und der Gaszelle möglichst zuverlässig vermieden wird.
Diese Aufgabe wird durch eine Vorrichtung zur Bestimmung der Konzentration zumindest eines Gases in einem Probengasstrom mittels Infrarotabsorptionsspektroskopie mit den Merkmalen des Hauptanspruchs gelöst. Die vorliegende Erfindung ist in Anspruch 1 definiert.

Dadurch, dass die Heizquelle innerhalb einer thermischen Isolierung der Gaszelle angeordnet ist, wobei innerhalb der thermischen Isolierung stromaufwärts eines Auslasses in den Raum ein durch die Heizquelle erwärmter Probengaskanal angeordnet ist, der sich in Längsrichtung der Gaszelle betrachtet zu beiden axialen Enden der Gaszelle erstreckt und zwei Auslässe aufweist, die jeweils in den Raum münden, wird erreicht, dass mit nur einer Heizquelle sowohl der Probengasstrom als auch die Gaszelle geheizt werden. So kann sichergestellt werden, dass zwischen dem In die Gaszelle einströmenden Probengasstrom und der Gaszelle kein Temperaturgradient auftritt. Dies verbessert die Messergebnisse und verringert den Aufwand zur Bereitstellung der Aufheizung und Temperaturüberwachung. Durch die zweiseitige Einleitung des Gases wird eine gleichmäßige Verteilung in der Gaszelle sichergestellt, was dazu führt, dass sehr schnell exakte Ergebnisse gewonnen werden können.

Vorzugsweise ist der Probengaskanal an einer zum Raum abgewandten Seite einer Begrenzungswand der Gaszelle ausgebildet. So wirken die Wände der Gaszelle als Wärmeleiter zur Aufheizung des Probengasstroms und des Inneren der Gaszelle, wodurch Temperaturunterschiede auf einfache Weise vermieden werden.

In einer hierzu weiterführenden Ausführung ist der Probengaskanal zumindest abschnittsweise durch eine Ausnehmung in der Begrenzungswand der Gaszelle gebildet. Eine solche Ausnehmung kann beispielsweise durch Fräsen in der Begrenzungswand hergestellt werden. Hierdurch wird ein direkter Wärmekontakt ohne Übergangsverluste zwischen dem Probengasstrom und der Gaszelle hergestellt.

In einer Weiterbildung teilt sich der Probengaskanal in zwei zu den Auslässen führende Teilkanäle auf, deren Lauflänge gleich ist. Dies stellt bei der Aufheizung des Probengasstroms in den Teilkanälen sicher, dass diese mit einer gleichen Endtemperatur in die Gaszelle strömen, da beide eine gleiche Wärmeübergangsfläche aufweisen.

Eine solche gleiche Lauflänge wird vorzugsweise dadurch sichergestellt, dass an der Begrenzungswand ein erster Probengaskanalabschnitt ausgebildet ist, der sich in Längsrichtung der Gaszelle betrachtet zu einer Mittelachse zwischen den beiden in Längsrichtung der Gaszelle betrachtet axialen Enden der Gaszelle erstreckt und sich der erste Probengaskanalabschnitt in Höhe der Mitte zwischen den in Längsrichtung der Gaszelle betrachtet axialen Enden in die zwei Teilkanäle aufteilt, die zu den axialen Enden führen. So wird mit einem einfach gestalteten Kanal eine maximale gleiche Lauflänge beider Teilkanäle sichergestellt. Mit dieser Lauflänge wird eine Temperatur des Probengasstroms beim Einströmen in die Gaszelle sichergestellt, die der Temperatur in der Gaszelle entspricht.

In einer alternativen Ausbildung der Erfindung ist der Probengaskanal mäanderförmig ausgebildet, wodurch die maximale Fläche der Begrenzungswand zum Aufheizen des Probengases genutzt werden kann.

In einer weiterführenden Ausführungsform ist der Probengaskanal am Boden der Gaszelle ausgebildet und durch eine Bodenplatte verschlossen. Entsprechend kann der Kanal durch Fräsen hergestellt und einfach durch den Deckel verschlossen werden. So wird einerseits eine sehr kostengünstige Herstellung sichergestellt und andererseits ein sehr guter Wärmeübergang zum Probengasstrom.

Weiterhin erstreckt sich der Probengaskanal vorteilhafterweise bis unmittelbar vor in Längsrichtung der Gaszelle betrachtet axiale Begrenzungswände der Gaszelle und die Auslässe münden jeweils in Längsrichtung der Gaszelle betrachtet auf der axialen Mittelachse der Gaszelle in den Raum. Auf diese Weise wird eine sehr gleichmäßige Verteilung des Probengasstroms in der Gaszelle von gegenüberliegenden Seiten sichergestellt, wodurch die Exaktheit der Messergebnisse erhöht wird.

Vorzugsweise münden die Auslässe des Probengaskanals in Längsrichtung der Gaszelle betrachtet axial zwischen den Objekt - oder Feldspiegeln und den axialen Begrenzungswänden in den Raum, so dass der Probengasstrom sich zunächst hinter den Spiegeln verteilt, bevor er in die eigentliche Messzone zwischen den Objekt- oder Feldspiegeln eintritt. Dies führt ebenfalls zu sehr guten Messergebnissen.

Des Weiteren ist es vorteilhaft, wenn die Heizquelle durch mehrere in den Seitenwänden der Gaszelle angeordnete elektrische Heizstäbe oder Heizmatten gebildet ist. Dies führt zu einer sehr gleichmäßigen Wärmeverteilung in den Wänden der Gaszelle, so dass Temperaturgradienten ausgeschlossen werden.

In einer hierzu weiterführenden Ausführung sind die Heizstäbe in senkrecht verlaufenden Bohrungen in den Seitenwänden angeordnet. Die Heizstäbe sind im eingebauten Zustand gut erreichbar, so dass sowohl der Anschluss der Heizstäbe als auch ein gegebenenfalls erforderlicher Austausch einfach durchzuführen sind.

Des Weiteren ist vorteilhafterweise in zumindest einer der Begrenzungswände ein Temperatursensor angeordnet, über den die Temperatur der Gaszelle gemessen wird. In Abhängigkeit der Messwerte dieses Sensors kann die zuzuführende Wärme geregelt werden. Der Sensor dient bei diesem Aufbau sowohl zur Überwachung der Probengastemperatur als auch der Gaszellentemperatur, so dass im Vergleich zu bekannten Ausführungen ein Sensor entfällt.

Zusätzlich sind die Begrenzungswände aus einem Material mit einer Wärmeleitfähigkeit von über 12 W/mK hergestellt, wodurch ein ausreichend guter Wärmeübergang zum Probengasstrom sichergestellt wird, so dass Temperaturunterscheide zwischen der Gaszelle und dem Probengasstrom auszuschließen sind.

Es wird somit eine Vorrichtung zur Bestimmung der Konzentration eines Gases in einem Probengasstrom mittels Infrarotabsorptionsspektroskopie geschaffen, mit der die Konzentration und Anwesenheit eines Gases mit hoher Genauigkeit und Reproduzierbarkeit festgestellt werden kann, da Temperaturgradienten vermieden werden. Durch die gemeinsame Regelung der Gaszellentemperatur und der Probengastemperatur vereinfacht sich zusätzlich der Aufbau durch den Entfall eines zusätzlichen Sensors, zusätzlicher Heizgeräte und deren Steuerungen, so dass die Vorrichtung kostengünstig hergestellt werden kann.

Ein Ausführungsbeispiel einer erfindungsgemäßen Vorrichtung zur Bestimmung der Konzentration zumindest eines Gases in einem Probengasstrom mittels Infrarotabsorptionsspektroskopie ist in der Figur anhand eines Analysators mit Quantenkaskadenlasers dargestellt und wird nachfolgend beschrieben.

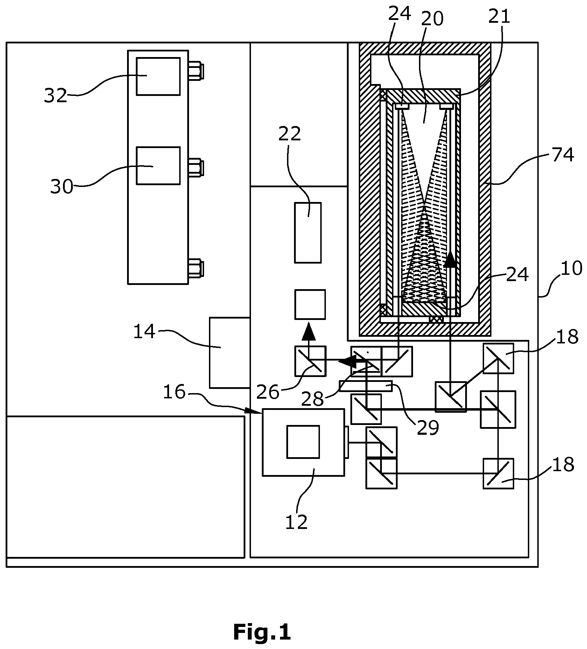

Die Figur 1 zeigt eine Prinzipdarstellung eines Quantenkaskadenlasers.

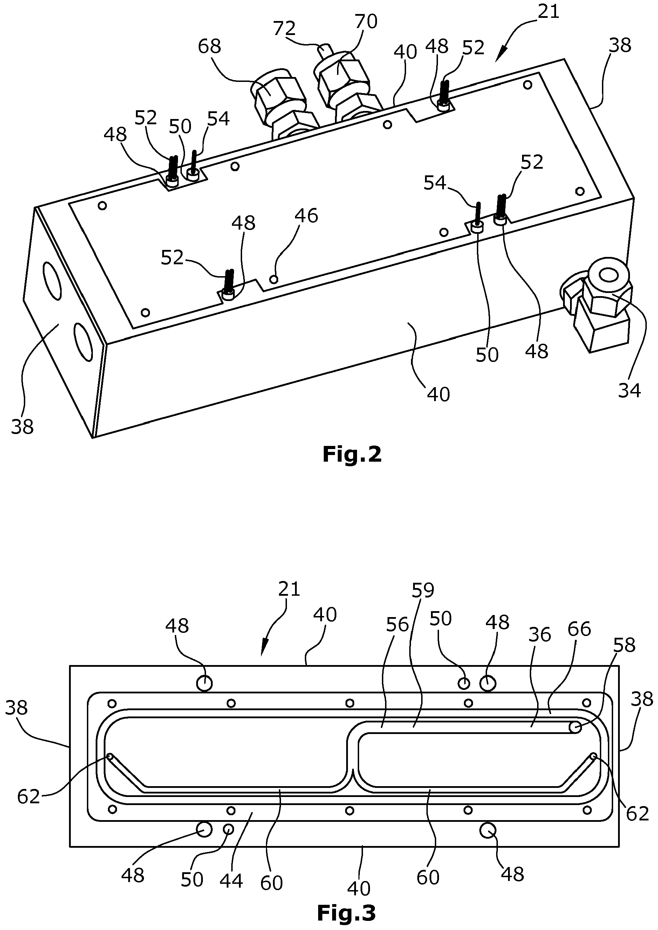

Die Figur 2 zeigt eine perspektivische Ansicht in das Innere einer Gaszelle.

Die Figur 3 zeigt eine Draufsicht auf den Boden der Gaszelle aus Figur 2.

Die erfindungsgemäße Vorrichtung zur Bestimmung der Konzentration zumindest eines Gases in einem Probengasstrom mittels Infrarotabsorptionsspektroskopie ist im vorliegenden Ausführungsbeispiel als Quantenkaskadenlaserabsorptionsspektrometer ausgeführt. Dieser besteht aus einem Gehäuse 10, in dem ein aus Halbleiterschichten aufgebauter Quantenkaskadenlaser 12 als Infrarotstrahlungsquelle angeordnet ist, der entweder kontinuierlich oder gepulst betrieben werden kann und insbesondere im mittleren Infrarotbereich Strahlung emittiert. Dieser wird über einen Stromtreiber 14 angesteuert und über ein Peltier-Element 16 gekühlt.

Der Strahl des Lasers 12 wird über mehrere Spiegel 18 in einen Raum 20 einer Gaszelle 21 oder alternativ über die Spiegel 18 direkt zu einem Detektor 22 geleitet, welcher beispielsweise ein MCT (Quecksilber-Cadmium-Tellurid)-Detektor sein kann, der besonders für die photovoltaische Detektion im mittleren Infrarotbereich geeignet ist und bei dem ein auftreffender Lichtquant direkt in einen messbaren Photostrom umsetzt. Im Raum 20 wird dieser Strahl mehrfach an Objekt- oder Feldspiegeln 24 reflektiert und durchdringt dabei ein in den Raum 20 gefördertes Probengas. Dies führt in bestimmten Frequenzbereichen des gesendeten Lichtbandes zu einer Absorption des Strahls, welche charakteristisch für die Anwesenheit und Konzentration bestimmter Moleküle ist. Nachdem der Strahl mehrfach an den Objekt- oder Feldspiegeln 24 reflektiert wurde, verlässt er die Gaszelle 21 wieder und wird erneut über folgende Spiegel 26 dem Detektor 22 zugeführt. Einer dieser Spiegel ist als Klappspiegel 28 ausgeführt, so dass je nach dessen Stellung entweder ein Referenzlaserstrahl über eine Referenzgasquelle 29 zum Detektor 22 gelangt oder der durch die Gaszelle 21 geführte Strahl.

Das vom Detektor 22 gemessene optische Frequenzband weist durch die absorbierte Strahlung Lücken auf, deren Größe und Tiefe ein Maß für die Konzentration des diesen Frequenzbereich absorbierenden Gases ist. Die entsprechende Umrechnung erfolgt in bekannter Weise über das Lambert-Beer-Gesetz. Die ausgesendete Wellenlänge des Lasers 12 kann dabei so eingestellt werden, dass selektiv der Absorptionsbereich einer bestimmten Absorptionslinie der Gaskomponente abgefahren werden kann, wodurch Querempfindlichkeiten zu anderen Gaskomponenten vermieden werden. So treten bei der Anwesenheit von Ammoniak beispielsweise Lücken im Wellenlängenbereich von etwa 10 µm auf.

Zu beachten ist jedoch, dass eine zuverlässige Messung nur bei einer korrekten Abstimmung zwischen der Weglänge des Strahls und der zu erwartenden Konzentration des zu messenden Moleküls im Probengasstrom möglich ist, so dass entweder mit einem unverdünnten oder einem verdünnten Probengasstrom gearbeitet werden muss. Zusätzlich müssen die Messbedingungen konstant gehalten werden. Insbesondere ist es erforderlich, die Temperatur des Probengasstromes und des Raums 20 beziehungsweise der Gaszelle 21 konstant zu halten, da die Absorption des Laserstrahls durch die Anregung der Moleküle erzeugt wird, welche sich mit ändernder Temperatur ebenfalls ändert. Dementsprechend können exakte Messergebnisse nur bei gleichen Temperaturbedingungen erzielt werden. Die optimale Temperatur ist dabei auch abhängig vom Verdünnungsgrad des Gases. Es hat sich gezeigt, dass Messungen bei einer konstanten Temperatur von 191°C bei einem Rohgas und von 60°C bei einem verdünnten Gas zu sehr guten Messergebnissen führen.

Die Probengasförderung erfolgt mittels einer Vakuumpumpe 30, mittels derer der Probengasstrom in den Raum 20 gesaugt wird. Der gesamte Strahlengang wird mit einem Gas, welches keine Moleküle des zu messenden Gases enthält, üblicherweise mit Stickstoff, gespült, um eine Verfälschung der Messergebnisse zu vermeiden.

An der Gaszelle 21 ist ein Probengaseinlassstutzen 34 ausgebildet, der über einen nicht dargestellten Schlauch mit einer Probengasquelle, wie beispielsweise einem Abgaskanal eines Verbrennungsmotors verbunden wird oder mit einer bereits verdünntes Probengas enthaltenden Quelle verbunden wird. Über die Vakuumpumpe 32 wird so das Probengas aus dem Einlassstutzen 34 über einen Probengaskanal 36 und den Raum 20 zur Vakuumpumpe 32 gesaugt.

Die Gaszelle 21 weist Im Wesentlichen eine Quaderform auf, so dass der Raum 20 durch sechs Begrenzungswände 38, 40, 42, 44 begrenzt ist. Zwei der Begrenzungswände dienen als Seitenwände 38 an den in Längsrichtung der Gaszelle 21 betrachtet axialen Enden hinter den Objekt- oder Feldspiegeln 24 und werden über Schrauben an den die seitliche Begrenzung bildenden Seitenwänden 40 befestigt. Zwischen diesen Seitenwänden 38 an den in Längsrichtung der Gaszelle betrachtet axialen Enden wird der Laserstrahl reflektiert. Nach oben wird die Gaszelle 21 durch einen als Begrenzungswand dienenden, auf die Seitenwände 38, 40 aufgeschraubten Deckel 42 begrenzt, nach unten durch einen Boden 44.
Neben den zur Befestigung des Deckels 42 notwendigen Bohrungen 46 in den Seitenwänden 40 sind an beiden Seitenwänden je drei weitere Bohrungen 48, 50 ausgebildet, die senkrecht zum Boden 44 verlaufen und nach oben hin offen sind. In je zwei dieser Bohrungen 48 sind als Heizquelle dienende elektrische Heizstäbe 52 angeordnet, die jeweils in einem Abstand von den axialen Seitenwänden 38 von etwa einem Viertel der Gesamtlänge der Seitenwände 40 angeordnet sind. Zusätzlich ist in jeder der Seitenwände 40 eine Bohrung 50 zur Aufnahme eines Temperatursensors 54 ausgebildet. Die Begrenzungswände 38, 40, 42, 44 sind aus Metall mit einer guten Wärmeleitfähigkeit von mindestens 12 W/mK hergestellt, um eine möglichst schnelle Erwärmung der Wände 38, 40, 42, 44 und des Raums 20 sicherstellen zu können.

An der zum Raum 20 abgewandten Seite des Bodens 44 ist der Probengaskanal 36 in Form einer Ausnehmung 56 an der Wandfläche eingefräst. Dieser verläuft vom Probengaseinlassstutzen 34 über eine Bohrung in der Seitenwand 40 zu einer Einlassöffnung 58 im Boden 44, welche sich im Bereich eines ersten axialen Endes der Gaszelle 21 befindet. Von hier verläuft der Probengaskanal 36 in einem ersten Probengaskanalabschnitt 59 parallel zur Seitenwand 40, bis er etwa in der Mitte zwischen den beiden axialen Seitenwänden 38 einen 90° Bogen vollzieht, so dass er sich im folgenden Verlauf zwischen den beiden seitlichen Seitenwänden 40 parallel zu den axialen Seitenwänden 38 erstreckt. Vor dem Erreichen der gegenüberliegenden Seitenwand 40 teilt sich der Probengaskanal 36 in zwei Teilkanäle 60 auf, die sich in entgegengesetzten 90° Bögen In einen weiterführenden gerade und parallel zu den Seitenwänden 40 verlaufenden Abschnitt der Teilkanäle erstrecken, so dass jeder Teilkanal in Richtung der axial begrenzenden Seitenwände 38 verläuft. Kurz vor dem Erreichen dieser Seitenwände 38 knicken die Teilkanäle 60 jeweils ab, so dass sie etwa auf der Mittelachse der Gaszelle 21 unmittelbar vor den Seitenwänden 38 in je einen Auslass 62 münden. Die Breite der Teilkanäle 60 entspricht etwa der Hälfte des vorgelagerten Probengaskanals 36, so dass durch den gleichen entstehenden Strömungswiderstand aufgrund gleicher Lauflängen und Querschnitte zwei etwa gleich große Teilströme entstehen, die über die beiden Auslässe 62 in den Messraum 20 strömen. Die Einströmung erfolgt dabei axial zwischen der axial begrenzenden Seitenwand und den vorgelagerten Objekt- oder Feldspiegeln 24, so dass eine Verteilung im Messraum 20 stattfindet, bevor das Probengas in den vom Laserstrahl durchleuchteten Bereich dringt. Ungenauigkeiten durch eine ungleichmäßige Verteilung des Probengasstroms oder Strömungsgeschwindigkeitsdifferenzen werden so vermieden. Der eingefräste Probengaskanal 36 wird durch eine verschraubte nicht dargestellte Bodenplatte verschlossen, wodurch der Probengaskanal 36 allseitig begrenzt wird. Selbstverständlich sind um den Probengaskanal 36 an der Auflagefläche der Bodenplatte wie auch an der Auflagefläche des Deckels 42 umlaufende Dichtungen 66 angeordnet.

An der zum Probengaseinlassstutzen 34 gegenüberliegenden Seitenwand 40 ist etwa in der Mitte zwischen den Seitenwänden 38 eine Auslassstutzen 68 angeordnet, über den der Probengasstrom wieder aus dem Raum 20 abgesaugt wird. Neben diesem Auslassstutzen 68 befindet sich ein Anschlussstutzen 70, in den ein Temperatursensor 72 eingesteckt ist, der in den Raum 20 ragt, um die dort vorhandene Temperatur zu messen.

Die gesamte Gaszelle 21 ist innerhalb einer thermischen Isolierung 74 angeordnet, durch die sichergestellt wird, dass im Raum 20 die gleiche Temperatur herrscht wie in den Begrenzungswänden 38, 40, 42, 44. Die Aufheizung der gesamten Gaszelle 21 und damit sowohl des Probengaskanals 36 als auch des Raums 20 erfolgt durch die gleiche Heizquelle, so dass der Probengasstrom und die Temperatur im Raum 20 gemeinsam gesteuert werden. Da der Probengaskanal im Boden lang genug gewählt ist, wird auch sichergestellt, dass das angesaugte Probengas vor dem Eintritt in den Raum 20 die gleiche Temperatur besitzt wie im Raum 20 herrscht. Somit werden durch die Verwendung eines einzigen Temperaturreglers Temperaturgradienten zwischen dem eingeführten Gas und dem Zelleninneren zuverlässig vermieden, wodurch sehr exakte Messergebnisse erzielt werden. Zusätzlich wird Energie durch den einen Aufwärmprozess ohne folgende Verluste über Leitungen eingespart.

Es sollte deutlich sein, dass die vorliegende Erfindung nicht auf das beschriebene Ausführungsbeispiel beschränkt ist, sondern verschiedene Modifikationen innerhalb des Schutzbereichs des vorliegenden Hauptanspruchs möglich sind. So kann der Kanal auch andersartig im Bereich der Gaszelle ausgebildet werden oder eine andere Form aufweise, wie beispielsweise eine Mäanderform. Auch sind konstruktive Änderungen der Gaszelle möglich. Diese Form der Erwärmung ist auch nicht auf die Verwendung an einem Quantenkaskadenlaser beschränkt sondern kann auch für andere Infrarotabsorptionsspektrometer genutzt werden.

## Patentansprüche

1. Vorrichtung zur Bestimmung der Konzentration zumindest eines Gases in einem Probengasstrom mittels Infrarotabsorptionsspektroskopie mit
einer Gaszelle (21) umfassend einen Raum (20), der sich entlang einer Achse in einer Längsrichtung der Gaszelle (21) erstreckt, einer Infrarotstrahlungsquelle (12), deren Strahlung durch den Raum (20) der Gaszelle (21) führbar ist, einem Probengaskanal (36) umfassend mindestens einen Auslass (62) in den Raum (20), über welchen ein Probengasstrom in den Raum (20) und die Strahlung leitbar ist,
einem Detektor (22), auf welchen die aus dem Raum (20) austretende Strahlung leitbar ist und mittels dessen das Absorptionsspektrum der austretenden Strahlung ermittelbar ist,
einer thermischen Isolierung (74) der Gaszelle (21), einer Heizquelle (52), über die der Probengasstrom auf eine Solltemperatur aufgeheizt wird, wobei die Heizquelle (52) innerhalb der thermischen Isolierung (74) der Gaszelle (21) angeordnet ist, wobei der Probengaskanal (36) innerhalb der thermischen Isolierung (74) stromaufwärts eines Auslasses (62) in den Raum (20) durch die Heizquelle (52) erwärmt wird, und der Probengaskanal (36) sich in Längsrichtung der Gaszelle (21) zu beiden axialen Enden der Gaszelle (21) erstreckt,
**dadurch gekennzeichnet, dass** der Probengaskanal (36) an beiden axialen Enden der Gaszelle (21) jeweils einen Auslass (62) aufweist, der in den Raum (20) der Gaszelle (21) mündet.

2. Vorrichtung zur Bestimmung der Konzentration zumindest eines Gases in einem Probengasstrom mittels Infrarotabsorptionsspektroskopie nach Anspruch 1,
**dadurch gekennzeichnet, dass**
der Probengaskanal (36) an einer zum Raum (20) abgewandten Seite einer Begrenzungswand (44) der Gaszelle (21) ausgebildet ist.

3. Vorrichtung zur Bestimmung der Konzentration zumindest eines Gases in einem Probengasstrom mittels Infrarotabsorptionsspektroskopie nach Anspruch 2,
**dadurch gekennzeichnet, dass**
der Probengaskanal (36) zumindest abschnittsweise durch eine Ausnehmung (56) in der Begrenzungswand (44) der Gaszelle (21) gebildet ist.

4. Vorrichtung zur Bestimmung der Konzentration zumindest eines Gases in einem Probengasstrom mittels Infrarotabsorptionsspektroskopie nach einem der vorhergehenden Ansprüche,
**dadurch gekennzeichnet, dass**
der Probengaskanal (36) sich in zwei Teilkanäle (60) aufteilt, wobei die Lauflänge beider zu den Auslässen (62) führenden Teilkanäle (60) gleich ist.

5. Vorrichtung zur Bestimmung der Konzentration zumindest eines Gases in einem Probengasstrom mittels Infrarotabsorptionsspektroskopie nach Anspruch 4,
**dadurch gekennzeichnet, dass**
an der Begrenzungswand (44) ein erster Probengaskanalabschnitt (59) ausgebildet ist, der sich zu einer Mittelachse zwischen den beiden in Längsrichtung der Gaszelle (21) betrachtet axialen Enden der Gaszelle (21) erstreckt und sich der erste Probengaskanalabschnitt (59) in Höhe der Mitte zwischen den in Längsrichtung der Gaszelle (21) betrachtet axialen Enden in die zwei Teilkanäle (60) aufteilt, die zu den axialen Enden führen.

6. Vorrichtung zur Bestimmung der Konzentration zumindest eines Gases in einem Probengasstrom mittels Infrarotabsorptionsspektroskopie nach einem der Ansprüche 1 bis 3,
**dadurch gekennzeichnet, dass**
der Probengaskanal (36) mäanderförmig ausgebildet ist.

7. Vorrichtung zur Bestimmung der Konzentration zumindest eines Gases in einem Probengasstrom mittels Infrarotabsorptionsspektroskopie nach einem der vorhergehenden Ansprüche,
**dadurch gekennzeichnet, dass**
der Probengaskanal (36) am Boden (44) der Gaszelle (21) ausgebildet Ist und durch eine Bodenplatte verschlossen ist.

8. Vorrichtung zur Bestimmung der Konzentration zumindest eines Gases in einem Probengasstrom mittels Infrarotabsorptionsspektroskopie nach Anspruch 7,
**dadurch gekennzeichnet, dass**
sich der Probengaskanal (36) in Längsrichtung der Gaszelle (21) betrachtet bis unmittelbar vor axiale Begrenzungswände (38) der Gaszelle (21) erstreckt und die Auslässe (62) jeweils auf der in Längsrichtung der Gaszelle (21) betrachtet axialen Mittelachse der Gaszelle (21) in den Raum (20) münden.

9. Vorrichtung zur Bestimmung der Konzentration zumindest eines Gases in einem Probengasstrom mittels Infrarotabsorptionsspektroskopie nach einem der Ansprüche 7 oder 8,
**dadurch gekennzeichnet, dass**
die Auslässe (62) des Probengaskanals (36) in Längsrichtung der Gaszelle (21) betrachtet axial zwischen Objekt- oder Feldspiegeln (24) und in Längsrichtung der Gaszelle (21) betrachtet axialen Begrenzungswänden (38) in den Raum (20) münden.

10. Vorrichtung zur Bestimmung der Konzentration zumindest eines Gases in einem Probengasstrom mittels Infrarotabsorptionsspektroskopie nach einem der vorhergehenden Ansprüche,
**dadurch gekennzeichnet, dass**
die Heizquelle durch eine in den Seitenwänden (40) der Gaszelle (21) angeordnete Heizmatte gebildet ist.

11. Vorrichtung zur Bestimmung der Konzentration zumindest eines Gases in einem Probengasstrom mittels Infrarotabsorptionsspektroskopie nach einem der vorhergehenden Ansprüche,
**dadurch gekennzeichnet, dass**
die Heizquelle durch mehrere in den Seitenwänden (40) der Gaszelle (21) angeordnete elektrische Heizstäbe (52) gebildet ist.

12. Vorrichtung zur Bestimmung der Konzentration zumindest eines Gases in einem Probengasstrom mittels Infrarotabsorptionsspektroskopie nach Anspruch 11,
**dadurch gekennzeichnet, dass**
die Heizstäbe (52) in senkrecht zur Längsrichtung der Gaszelle (21) verlaufenden Bohrungen (48) in den Seitenwänden (40) angeordnet sind.

13. Vorrichtung zur Bestimmung der Konzentration zumindest eines Gases in einem Probengasstrom mittels Infrarotabsorptionsspektroskopie nach einem der vorgehenden Ansprüchen,
**dadurch gekennzeichnet, dass**
in zumindest einer der Begrenzungswände (38, 40, 42, 44) der Gaszelle (21) ein Temperatursensor (54) angeordnet ist.

14. Vorrichtung zur Bestimmung der Konzentration zumindest eines Gases in einem Probengasstrom mittels Infrarotabsorptionsspektroskopie nach einem der vorgehenden Ansprüchen,
**dadurch gekennzeichnet, dass**
die Begrenzungswände (38, 40, 42, 44) der Gaszelle (21) aus einem Material mit einer Wärmeleitfähigkeit von über 12 W/mK hergestellt sind.

## Claims

1. A device for determining the concentration of at least one gas in a sample gas flow by means of infrared absorption spectroscopy, comprising
a gas cell (21) with a chamber (20) that extends along an axis in the longitudinal direction of the gas cell (21),
an infrared radiation source (12) the radiation of which is adapted to be conducted through the chamber (20) of the gas cell (21),
a sample gas duct (36) comprising at least one outlet (62) into the chamber (20) via which a sample gas flow can be conducted into the chamber (20) and the radiation,
a detector (22) to which the radiation exiting said chamber (20) is adapted to be conducted and by means of which the absorption spectrum of the exiting radiation is adapted to be determined,
a thermal insulation (74) of the gas cell (21),
a heating source (52) via which the sample gas flow is heated to a desired temperature, wherein said heating source (52) is arranged within the thermal insulation (74) of said gas cell (21), wherein within said thermal insulation (74) upstream of the outlet (62) into said chamber (20), the sample gas duct (36) is heated by said heating source (52), and the sample gas duct (36) extends in the longitudinal direction of the gas cell (21) to the two axial ends of the gas cell (21),
**characterized in that**
the sample gas duct (36) has a respective outlet (62) at each of the two axial ends of the gas cell (21), which opens into the chamber (20) of the gas cell (21).

2. The device for determining the concentration of at least one gas in a sample gas flow by means of infrared absorption spectroscopy according to claim 1,
**characterized in that**
the sample gas duct (36) is defined at a side of a boundary wall (44) of the gas cell (21) facing away from the chamber (20).

3. The device for determining the concentration of at least one gas in a sample gas flow by means of infrared absorption spectroscopy according to claim 2,
**characterized in that**
at least sections of the sample gas duct (36) are defined by a recess (56) in the boundary wall (44) of the gas cell (21).

4. The device for determining the concentration of at least one gas in a sample gas flow by means of infrared absorption spectroscopy according to any one of the preceding claims,
**characterized in that**
the sample gas duct (36) branches into two sub-ducts (60), wherein the run lengths of the two sub-ducts (60) extending to the outlets (62) are the same.

5. The device for determining the concentration of at least one gas in a sample gas flow by means of infrared absorption spectroscopy according to claim 4,
**characterized in that**
at the boundary wall (44) a first sample gas duct section (59) is defined which extends to a central axis between the two axial ends of the gas cell (21), seen in the longitudinal direction, and the first sample gas duct section (59) is divided into the two sub-ducts (60) at the level of the middle between the axial ends, seen in the longitudinal direction, said sub-ducts extending to the axial ends.

6. The device for determining the concentration of at least one gas in a sample gas flow by means of infrared absorption spectroscopy according to any one of claims 1 to 3,
**characterized in that**
the sample gas duct (36) is of a meander-shaped configuration.

7. The device for determining the concentration of at least one gas in a sample gas flow by means of infrared absorption spectroscopy according to any one of the preceding claims,
**characterized in that**
the sample gas duct (36) is defined at the base (44) of the gas cell (21) and is closed by a base plate.

8. The device for determining the concentration of at least one gas in a sample gas flow by means of infrared absorption spectroscopy according to claim 7,
**characterized in that**
the sample gas duct (36) extends up to a place immediately in front of axial boundary walls (38) of the gas cell (21), seen in the longitudinal direction of the gas cell (21), and the outlets (62) each enter the chamber (20) on the axial central axis of said gas cell (21), seen respectively in the longitudinal direction of the gas cell (21).

9. The device for determining the concentration of at least one gas in a sample gas flow by means of infrared absorption spectroscopy according to claim 7 or 8,
**characterized in that**
seen in the longitudinal direction of the gas cell (21), the outlets (62) of the sample gas duct (36) enter the chamber (20) axially between object or field mirrors (24) and, seen in the longitudinal direction of the gas cell (21), axial boundary walls (38).

10. The device for determining the concentration of at least one gas in a sample gas flow by means of infrared absorption spectroscopy according to any one of the preceding claims,
**characterized in that**
the heating source is defined by a heating mat arranged in the side walls (40) of the gas cell (21).

11. The device for determining the concentration of at least one gas in a sample gas flow by means of infrared absorption spectroscopy according to any one of the preceding claims,
**characterized in that**
the heating source is defined by a plurality of electric heating rods (52) arranged in the side walls (40) of the gas cell (21).

12. The device for determining the concentration of at least one gas in a sample gas flow by means of infrared absorption spectroscopy according to claim 11,
**characterized in that**
the heating rods (52) are arranged in bores (48) in the side walls (40), said bores extending vertically with respect to the longitudinal direction of the gas cell (21).

13. The device for determining the concentration of at least one gas in a sample gas flow by means of infrared absorption spectroscopy according to any one of the preceding claims,
**characterized in that**
in at least one of the boundary walls (38, 40, 42, 44) of the gas cell (21) a temperature sensor (54) is arranged.

14. The device for determining the concentration of at least one gas in a sample gas flow by means of infrared absorption spectroscopy according to any one of the preceding claims,
**characterized in that**
the boundary walls (38, 40, 42, 44) of the gas cell (21) are made of a material with a heat conductivity of more than 12 W/mK.

## Revendications

1. Dispositif pour déterminer la concentration d'au moins un gaz dans un flux de gaz échantillon par spectroscopie d'absorption infrarouge comprenant
une cellule à gaz (21) comprenant une chambre (20) s'étendant le long d'un axe dans une direction longitudinale de la cellule de gaz (21),
une source de rayonnement infrarouge (12), dont le rayonnement peut être dirigé à travers la chambre (20) de ladite cellule à gaz (21),
un canal de gaz échantillon (36) comprenant au moins une sortie (52) dans la chambre (20), à travers lequel un flux de gaz échantillon peut être conduit dans l'chambre (20) et le rayonnement,
un détecteur (22), vers lequel le rayonnement sortant de la chambre (20) peut être dirigé et à l'aide duquel le spectre d'absorption du rayonnement émergeant peut être déterminé,
une isolation thermique (74) de la cellule à gaz (21),
une source de chauffage (52) par laquelle le flux de gaz échantillon est chauffé à une température préréglée, la source de chauffage (52) étant disposée au sein de l'isolation thermique (74) de la cellule à gaz (21), ledit canal de gaz échantillon (36) étant chauffé par la source de chauffage (52= au sein de l'isolation thermique (74) et en amont d'une sortie (62) dans la chambre (20), et le canal de gaz échantillon (36) s'étendant dans la direction longitudinale de la cellule à gaz (21) vers les deux extrémités axiales de la cellule à gaz (21),
**caractérisé en ce que**
le canal de gaz échantillon (36) comprend une sortie (62) respective aux deux extrémités de la cellule à gaz (21), lesdites sorties s'ouvrant dans la chambre (20) de la cellule à gaz (21).

2. Dispositif pour déterminer la concentration d'au moins un gaz dans un flux de gaz échantillon par spectroscopie d'absorption infrarouge selon la revendication 1, **caractérisé en ce que** le canal de gaz échantillon (36) est formé sur un côté d'une paroi limite (44) de la cellule à gaz (21) détournée de la chambre (20).

3. Dispositif pour déterminer la concentration d'au moins un gaz dans un flux de gaz échantillon par spectroscopie d'absorption infrarouge selon la revendication 2, **caractérisé en ce que** le canal de gaz échantillon (36) est formé au moins en sections par un évidement (56) dans la paroi limite (44) de la cellule à gaz (21).

4. Dispositif pour déterminer la concentration d'au moins un gaz dans un flux de gaz échantillon par spectroscopie d'absorption infrarouge selon l'une quelconque des revendications précédentes, **caractérisé en ce que** le canal de gaz échantillon (36) se sépare en deux canaux partiels (60).
la longueur de course des deux canaux partiels (60) menant aux sorties (62) est la même.

5. Dispositif pour déterminer la concentration d'au moins un gaz dans un flux de gaz échantillon par spectroscopie d'absorption infrarouge selon la revendication 4, **caractérisé en ce qu'**une première section de canal de gaz échantillon (59) est formée sur la paroi limite (44), qui s'étend jusqu'à un axe central entre les deux extrémités axiales de la cellule à gaz (21), vu dans la direction longitudinale de la cellule à gaz (21), et la première section de canal de gaz échantillon (59) se sépare au niveau du milieu entre les extrémités axiales, vu dans la direction longitudinale de la cellule à gaz (21), en les deux sous-canaux (60) menant aux extrémités axiales.

6. Dispositif pour déterminer la concentration d'au moins un gaz dans un flux de gaz échantillon par spectroscopie d'absorption infrarouge selon l'une des revendications 1 à 3, **caractérisé en ce que** le canal de gaz échantillon (36) est sinueux.

7. Dispositif pour déterminer la concentration d'au moins un gaz dans un flux de gaz échantillon par spectroscopie d'absorption infrarouge selon l'une quelconque des revendications précédentes, **caractérisé en ce que** le canal de gaz échantillon (36) est formé sur le fond (44) de la cellule à gaz (21) et est fermé par une plaque de fond.

8. Dispositif pour déterminer la concentration d'au moins un gaz dans un courant de gaz échantillon par spectroscopie d'absorption infrarouge selon la revendication 7, **caractérisé en ce que**, vu dans la direction longitudinale de la cellule à gaz (21), le canal de gaz échantillon (36) s'étend immédiatement devant les parois limites axiales (38) de la cellule à gaz (21) et chacune des sorties (62) s'ouvre dans la chambre (21) sur la l'axe central axial de la cellule à gaz (21), vu dans la direction longitudinale de la cellule à gaz (21).

9. Dispositif pour déterminer la concentration d'au moins un gaz dans un flux de gaz échantillon par spectroscopie d'absorption infrarouge selon l'une des revendications 7 ou 8, **caractérisé en ce que** les sorties (62) du canal de gaz échantillon (36) s'ouvrent dans la chambre (20), vu dans la direction longitudinale de la cellule à gaz (21), axialement entre des miroirs d'objet ou de champ (24) et les parois limites axiales (38), vu dans la direction longitudinale de la cellule à gaz (21).

10. Dispositif pour déterminer la concentration d'au moins un gaz dans un flux de gaz échantillon par spectroscopie d'absorption infrarouge selon l'une quelconque des revendications précédentes, **caractérisé en ce que** la source de chauffage est formée par un coussin chauffant disposé dans les parois latérales (40) de la cellule à gaz (21).

11. Dispositif pour déterminer la concentration d'au moins un gaz dans un flux de gaz échantillon par spectroscopie d'absorption infrarouge selon l'une quelconque des revendications précédentes, **caractérisé en ce que** la source de chauffage est formée par plusieurs éléments chauffants électriques (52) disposés dans les parois latérales (40) de la cellule à gaz (21).

12. Dispositif pour déterminer la concentration d'au moins un gaz dans un flux de gaz échantillon par spectroscopie d'absorption infrarouge selon la revendication 11, **caractérisé en ce que** les éléments chauffants (52) sont disposées dans des trous verticaux (48), vu par rapport à la direction longitudinale de la cellule à gaz (21), dans les parois latérales (40).

13. Dispositif pour déterminer la concentration d'au moins un gaz dans un flux de gaz échantillon par spectroscopie d'absorption infrarouge selon l'une quelconque des revendications précédentes, **caractérisé en ce qu'**un capteur de température (54) est disposé dans au moins une des parois limites (38, 40, 42, 44) de la cellule à gaz (21).

14. Dispositif pour déterminer la concentration d'au moins un gaz dans un flux de gaz échantillon par spectroscopie d'absorption infrarouge selon l'une quelconque des revendications précédentes, **caractérisé en ce que** les parois limites (38, 40, 42, 44) de la cellule à gaz (21) sont formées d'un matériau avec une conductivité thermique supérieure à 12 W/mK.
